# EUROPEAN PATENT APPLICATION

(11) **EP 1 086 708 A2**
(43) Date of publication of application: **28.03.2001**
(21) Application number: 00118651.9
(22) Date of filing: 29.08.2000
(51) Int. Cl.: A61L 2/07

(54) **Sterilization-in-place arrangement and method for intravenous bags and the like**

(30) Priority: 27.09.1999 US 406587
(71) Applicant: Saint-Gobain Performance Plastics Corporation, Wayne, New Jersey 07470 (US)
(72) Inventor: Warburton-Pitt, Stephen, Andover, New Jersey 07821 (US)
(74) Representative: DIEHL GLAESER HILTL & PARTNER

(57) **Abstract**

An arrangement and method for the sterilization-in-place of a bag assembly includes a bypass tube connected to the inlet and outlet tubes of the bag assembly. Clamps on the inlet and outlet tubes and on the bypass tube are utilized to selectively route pressurized steam used for sterilization and solution for filling the bag.

## Description

### BACKGROUND OF THE INVENTION

This invention relates to a bag for use in pharmaceutical manufacturing and for holding health care related solutions and, more particularly, to an arrangement and method for use in the sterilization-in-place of such a bag.

Bag assemblies including a flexible bag and tubing connections are currently used in many applications, including intravenous delivery, blood transfusion and pharmaceutical manufacture. Before being filled, the bag assemblies must be sterilized. This is conventionally done initially at the place of manufacture before the bag assemblies are shipped, and the sterile bag assemblies are placed within individual packaging which protects their sterility. When a bag assembly reaches the location where it is to be filled, and the outer package is opened, unless this is done in a sterile environment, the bag assembly loses its sterility. Usually, clamps are applied to the tubes so that the interior of the bag remains sterile, but the interiors of the tubes distally of the clamps lose their sterility. This interior then has to be resterilized before the bag can be filled. It would therefore be desirable to provide an arrangement and method whereby the bag tube interiors can be sterilized in place immediately before the bag is filled and at the same station where the bag is filled.

### SUMMARY OF THE INVENTION

According to the present invention, in order to effect sterilization-in-place of a bag assembly having a bag secured to a pair of bag tubes, each bag tube communicating with the interior of the bag, there is provided a bypass manifold and a plurality of clamp members. The bypass manifold includes a first tube having a first end adapted to receive pressurized steam and a second end adapted for connection to a first of the pair of bag tubes, a second tube having a first end adapted for connection to the second of the pair of bag tubes and a second end, and a bypass tube connected to the first tube between its first and second ends and to the second tube between its first and second ends. The plurality of clamp members are adapted to seal respective selected sites on the tubes.

In accordance with an aspect of this invention, the bypass manifold is formed unitarily with the pair of bag tubes. A first three-way "T" connector joins the first bag tube to the first tube and to a first end of the bypass tube. A second three-way "T" connector joins the second bag tube to the second tube and to the second end of the bypass tube.

### BRIEF DESCRIPTION OF THE DRAWINGS

The foregoing will be more readily apparent upon reading the following description in conjunction with the drawings in which like elements in different figures thereof are identified by the same reference numeral and wherein:
FIGURE 1 is a plan view of a first embodiment of a bag assembly and bypass manifold constructed according to the principles of this invention;
FIGURE 2 is an elevational view showing an illustrative clamp member mounted on a tube; and
FIGURE 3 is a plan view of a second embodiment of a bag assembly and bypass manifold constructed according to the present invention.

### DETAILED DESCRIPTION

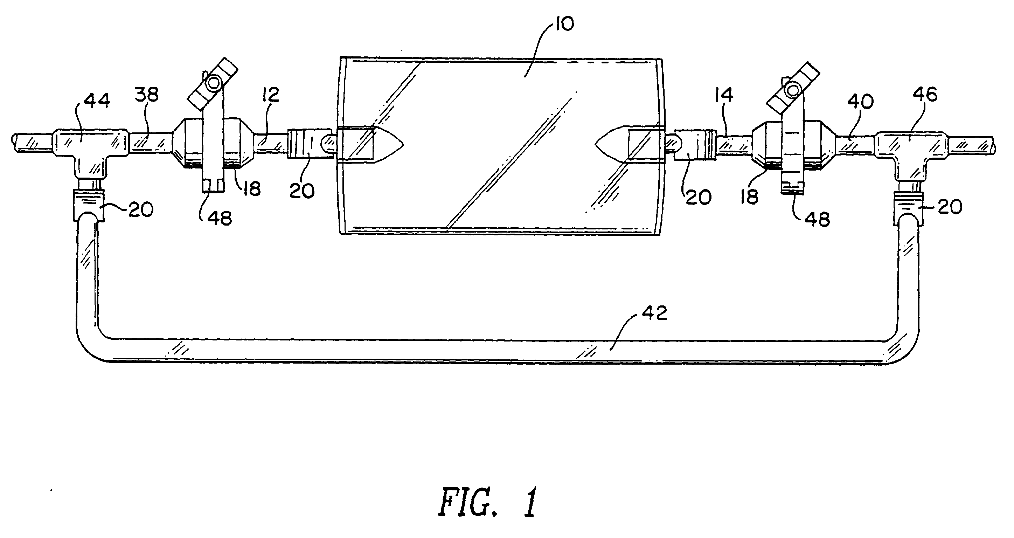

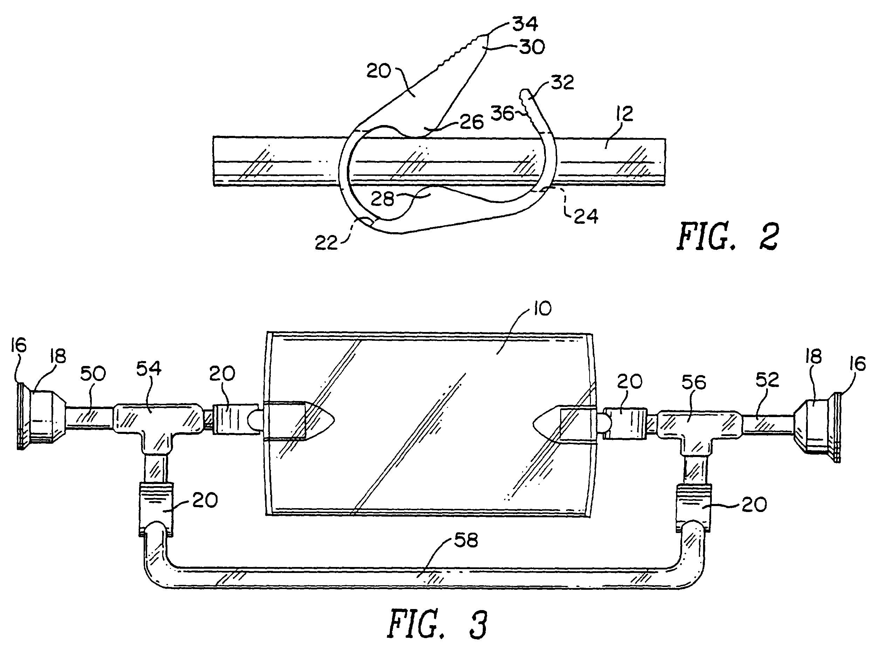

Referring now to the drawings, Figure 1 shows a first embodiment of a bag assembly and a bypass manifold used for sterilization-in-place of the bag assembly. The bag assembly includes a bag 10 secured to a pair of bag tubes 12, 14 each communicating with the interior of the bag 10. Preferably, the bag 10 and the tubes 12, 14 are formed from silicone tubing, with the bag 10 being formed from large bore, thin walled silicone tubing and the tubes 12, 14 being formed from small bore, thick walled silicone tubing. Other suitable materials, such as PVC for example, can also be used for the bag assembly. The tubes 12, 14 are inserted into the ends of the tubing forming the bag 10, and the ends of the bag tubing are then flattened and sealed to themselves and to the tubes 12, 14. The distal ends of the tubes 12, 14 are formed with silicone flanges 16 and have slidingly mounted thereon stainless backup cups 18, as best shown in Figure 3.

When the bag assembly including the bag 10 and the tubes 12, 14 is manufactured, before each of the tubes 12, 14 are inserted into the end of the open bag 10, a clamp member 20 is placed on each of the tubes 12, 14. As shown in Figure 2, the clamp member 20 mounted on the tube 12 is a unitary structure having openings 22, 24 therethrough to allow the tube 12 to be inserted. The clamp member 20 is formed with a pair of opposed projections 26, 28. When the end 30 of the clamp member is moved toward the end 32, the projections 26, 28 squeeze the tube 12 therebetween. Upon application of sufficient force to the end 30, which can be done manually, the projections 26, 28 are moved sufficiently close together that the tube 12 is flattened and its central bore is closed off. The end 30 is pointed, at 34, and the end 32 has serrations 36 which hold the point 34 to maintain the clamp member 20 in its sealing condition. Moving the end 32 clockwise, as viewed in Figure 2, releases the point 34 from the serrations 36 and opens the tube 12.

According to the present invention, a bypass is provided around the inlet and outlet tubes 12, 14. In accordance with a first embodiment of the present invention, as shown in Figure 1, this bypass is provided by a bypass manifold including a first tube 38, a second tube 40, and a bypass tube 42. The tubes 38, 40 are terminated the same way as the tubes 12, 14 (i.e., with a flange and a stainless steel backup cup at each end). The bypass tube 42 is connected to the tube 38 between its first and second ends and to the tube 40 between its first and second ends. Preferably, the bypass tube 42 is formed unitarily with the tubes 38, 40, with a first three-way "T" connector joining the bypass tube 42 to the tube 38 and a second three-way "T" connector 46 joining the bypass tube 42 to the tube 40. The bypass tube 42 is also provided with a pair of clamp members 20 each adjacent a respective "T" connector 44, 46.

To perform sterilization-in-place of the bag assembly shown in Figure 1 with the bypass manifold comprising the tubes 38, 40, 42, the bag assembly including the bag 10 and the bag tubes 12, 14 is removed from its outer packaging after the clamps 20 have sealed the tubes 12, 14. This causes the tubes 12, 14 to become unsterile distally of the clamps 20, but preserves the sterility of the interior of the bag 10 and the interior of the tubes 12, 14 up to the clamps 20. The bypass manifold is then secured to the fittings of the tubes 12, 14 by the clamps 48, which grip the backup cups while holding the tube flanges in abutting relationship. The assembly is then transported to a sterilization station, which preferably is part of a bag filling station. The fitting (not shown) at the free end of one of the tubes 38, 40 is then connected to the sterilization station and pressurized steam is provided. Pressurized steam is a known, and approved, sterilization technique. Assuming that it is the tube 38 that is connected to the source of pressurized steam, the pressurized steam travels through the tube 38 and into the tube 12 up to where the clamp 20 has sealed the tube 12. The clamps 20 on the bypass tube 42 remain open, so that the pressurized steam travels through the "T" connector 44 to the bypass tube 42, and at the "T" connector 46, the pressurized steam travels through the tube 40, passing into the tube 14 up to where the clamp 20 has sealed off the tube 14, the remainder of the pressurized steam exiting the free end (not shown) of the tube 40. This process sterilizes the interior of the tubes 38, 40, 42, as well as those portions of the tubes 12, 14 distally of the bag 10. Keeping the tube 38 in place, the steaming is terminated, and the clamps 20 on the tube 42 are closed and the clamps 20 on the tubes 12, 14 are opened. Material from the filling station is then supplied to the tube 38. This material passes through the tube 38 to the tube 12 and into the bag 10. The material exits the bag 10 through the tube 14 and the tube 40. The clamp 20 on the tube 14 is then closed and the bag 10 is filled with the appropriate amount of material. The clamp 20 on the tube 12 is then closed. The tube 38 is then removed from the filling station and the clamps 48 are opened to release the bypass manifold from the bag assembly. The bypass manifold may then be discarded and the bag assembly containing liquid material may then be transported to its ultimate destination.

Figure 3 shows an alternate embodiment of the present invention wherein the inlet and outlet tubes of the bag 10 are formed unitarily with the bypass tube. As shown, the inlet and outlet tubes 50, 52 are formed integrally with three-way "T" connectors 54, 56, respectively, which are joined to opposite ends of the bypass tube 58. Clamps 20 are provided on the tubes 50, 52 between the "T" connectors 54, 56 and the bag 10, and clamps 20 are also provided on the bypass tube 58 adjacent the "T" connectors 54, 56. The assembly shown in Figure 3 is used identically with the assembly shown in Figure 1, as described above, with the exception that the bypass tube 58 is not discarded after use.

Accordingly, there has been disclosed an improved sterilization-in-place arrangement and method for a bag assembly. While illustrative embodiments of the present invention have been disclosed herein, it is understood that various modifications and adaptations to the disclosed embodiments are possible and it is intended that this invention be limited only by the scope of the appended claims.

## Claims

1. An arrangement for use in performing sterilization-in-place of a bag assembly having a bag secured to a pair of bag tubes each communicating with the interior of the bag, comprising:
a bypass manifold including:
a first tube having a first end adapted to receive pressurized steam and a second end adapted for connection to a first of the pair of bag tubes;
a second tube having a first end adapted for connection to the second of the pair of bag tubes and a second end; and
a bypass tube connected to said first tube between its first and second ends and to said second tube between its first and second ends; and
a plurality of clamp members adapted to seal respective selected sites on said tubes.

2. The arrangement according to Claim 1 wherein said plurality of clamp members comprises:
a first clamp member adapted to seal said first tube;
a second clamp member adapted to seal said second tube; and
at least one third clamp member adapted to seal said bypass tube.

3. The arrangement according to Claim 2 wherein each of said clamp members comprises a unitary member slidingly mounted on its respective tube and having a pair of opposed projections adapted to be moved toward each other to squeeze the respective tube therebetween.

4. The arrangement according to Claim 1 wherein said bypass tube is formed unitarily with said first and second tubes, with a first three-way "T" connector joining the first tube to a first end of the bypass tube and a second three-way "T" connector joining the second tube to the second end of the bypass tube.

5. The arrangement according to Claim 1 wherein said bypass manifold is formed unitarily with said pair of bag tubes, with a first three-way "T" connector joining the first bag tube to the first tube and to a first end of the bypass tube and a second three-way "T" connector joining the second bag tube to the second tube and to the second end of the bypass tube.

6. The arrangement according to Claim 5 wherein said plurality of clamp members comprises:
a first clamp member adapted to seal said first bag tube;
a second clamp member adapted to seal said second bag tube; and
at least one third clamp member adapted to seal said bypass tube.

7. The arrangement according to Claim 6 wherein each of said clamp members comprises a unitary member slidingly mounted on its respective tube and having a pair of opposed projections adapted to be moved toward each other to squeeze the respective tube therebetween.

8. A method for performing sterilization-in-place of a bag assembly having a bag secured to a pair of bag tubes each communicating with the interior of the bag, comprising the steps of:
a) providing a bypass manifold including:
a first tube having a first end adapted to receive pressurized steam and a second end adapted for connection to a first of the pair of bag tubes;
a second tube having a first end adapted for connection to the second of the pair of bag tubes and a second end; and
a bypass tube connected to said first tube between its first and second ends and to said second tube between its first and second ends;
b) providing a plurality of clamp members adapted to seal respective selected sites on said bag tubes and said bypass tube;
c) connecting the second end of the first tube to the first bag tube;
d) connecting the first end of the second tube to the second bag tube;
e) closing the clamps on the pair of bag tubes; and
f) providing pressurized steam to the first tube.

9. The method according to Claim 8 further comprising the steps of:
g) removing the pressurized steam;
h) closing the clamps on the bypass tube;
i) opening the clamps on the pair of bag tubes;
j) providing material for filling the bag to the first tube; and
k) closing the clamps on the pair of bag tubes.
